(19) Europäisches Patentamt
European Patent Office
Office européen des brevets

(11) Veröffentlichungsnummer: **0 312 726 B1**

# (12) EUROPÄISCHE PATENTSCHRIFT

(45) Veröffentlichungstag der Patentschrift: **08.04.92**

(51) Int. Cl.⁵: **C07D 277/06**, C07C 215/40

(21) Anmeldenummer: **88113190.8**

(22) Anmeldetag: **13.08.88**

Die Akte enthält technische Angaben, die nach dem Eingang der Anmeldung eingereicht wurden und die nicht in dieser Patentschrift enthalten sind.

## (54) Optisch aktive Salze aus einem substituierten Thiazolidin-4-carboxylat und 3-Chlor-2-hydroxypropyltrimethylammonium, deren Herstellung und Verwendung.

(30) Priorität: **22.10.87 DE 3735757**

(43) Veröffentlichungstag der Anmeldung:
**26.04.89 Patentblatt 89/17**

(45) Bekanntmachung des Hinweises auf die Patenterteilung:
**08.04.92 Patentblatt 92/15**

(84) Benannte Vertragsstaaten:
**AT BE CH DE ES FR GB IT LI NL SE**

(56) Entgegenhaltungen:
**EP-A- 0 157 315**
**US-A- 3 947 464**

(73) Patentinhaber: **Degussa Aktiengesellschaft**
**Weissfrauenstrasse 9**
**W-6000 Frankfurt am Main 1(DE)**

(72) Erfinder: **Jakob, Harald, Dr.**
**Hauptstrasse 78**
**W-6467 Hasselroth(DE)**
Erfinder: **Huthmacher, Klaus, Dr.**
**Lärchenweg 18**
**W-6460 Gelnhausen(DE)**
Erfinder: **Klenk, Herbert, Dr.**
**Gleiwitzer Strasse 21**
**W-6450 Hanau(DE)**
Erfinder: **Kleemann, Axel, Prof. Dr.**
**Bornweg 36**
**W-6052 Mühlheim/Main(DE)**
Erfinder: **Giray, Gunes**
**Goethestrasse 71**
**W-8752 Kleinostheim(DE)**

EP 0 312 726 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft neue optisch aktive Salze aus einem substituierten Thiazolidin -4-carboxylat und 3-Chlor-2-hydroxypropyltrimethylammonium und deren Verwendung zur Herstellung eines optisch aktiven 3-Chlor-2-hydroxypropyltrimethylammoniumchlorids (Quab®188). Bei der L-(-)-Verbindung handelt es sich um einen wertvollen Baustein für die Synthese von L-(-)-Carnitin.

Dessen Darstellung erfolgte bislang via Racematspaltung der entsprechenden Vorstufen Carnitinnitrilchlorid und Carnitinamidchlorid, wobei zunächst mit Silbersalzen (DD-PS 23 217), Anionenaustauscher (DE-OS 2 927 672) oder Elektrodialyse (DE-OS 3 342 713) das Chlorid-Ion entfernt und gegen ein optisch aktives Carboxylat ausgetauscht werden muß.

Der Nachteil dieser Verfahren liegt nicht nur in den großen Mengen anfallender, schwer abtrennbarer Salzverunreinigungen, die die Racematspaltung erschweren, sondern auch in der Anhäufung des nicht racemisierbaren und somit nicht wieder verwertbaren D-Enantiomeren, was besonders ökologische Probleme aufwirft.

Erfolgt die Racematspaltung jedoch auf einer frühen Vorstufe (z. B. von Quab®188) sind die weiteren Syntheseschritte bis zu L-Carnitin nicht mehr durch die unerwünschten enantiomeren Verbindungen belastet. Dies ist für die Wirtschaftlichkeit der Synthese von großer Bedeutung. Eine Verwendung des hier unbrauchbaren Enantiomeren D-(+)-Quab zu ansonsten für Quab bekannten Zwecken erscheint zudem denkbar.

Aus der EP-A-0157315 ist ein Verfahren zur Herstellung des optisch aktiven Di-(3-chlor-2-hydroxypropyltrimethylammonium)-tatras bekannt, aus dem man die gewünschte Verbindung durch Abtrennen des Tatrats gewinnen kann.

Die Weinsäure läßt jedoch als Spaltreagens für technische Zwecke zu wünschen übrig, da sie sich wegen ihrer hohen Wasserlöslichkeit aus den wässrigen Lösungen, die man bei der Freisetzung des Quabs 188 aus dem entsprechenden Salzpaar erhält, nur mit großem Aufwand zurückgewinnen läßt. Zudem ist wegen des geringen Löslichkeitsunterschieds von Quab 188 und Weinsäure in Wasser sowie organischen Lösungsmitteln die Ausbeute an freigesetztem optisch aktiven Quab nicht befriedigend.

Der Erfindung lag daher die Aufgabe zugrunde, Quab 188 auf einfachere und wirtschaftlichere Weise in seine optisch Antipoden zu spalten als bisher.

Gegenstand der Erfindung sind optisch aktive Salze der allgemeinen Formel

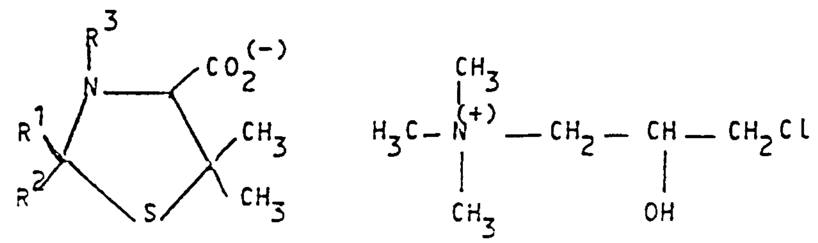

(I)

in der bedeuten

$R^1$, $R^2$, gleich oder verschieden: Wasserstoff, Alkyl mit 1 bis 8 C-Atomen, insbesondere Methyl und Ethyl,

$R^1$ oder $R^2$: Cycloalkyl mit 4 bis 11 C-Atomen

$R^3$ eine Acylgruppe, insbesondere Benzoyl, Tosyl, Nitrophenylsulfenyl, Acetyl oder Formyl.

Als bevorzugte Ausführungsformen sind anzusehen, das Satz aus D-(+)-3-Formyl-2,2,5,5,-tetramethylthiazolidin-4-carboxylat und L-(-)-3-Chlor-2-hydroxypropyltrimethylammonium und das Salz aus L-(-)-3-Formyl-2,2,5,5-tetramethylthiazolidin-4-carboxylat und D-(+)-3-Chlor-2-hydroxypropyltrimethylammonium.

Ein erfindungsgemäßes Verfahren zur Herstellung der optisch aktiven Salze gemäß der allgemeinen Formel (I) bei dem man eine optisch aktive Säure mit Trimethylamin zu einem Trimethylammoniumsalz umsetzt, das man anschließend mit racemischem Epichlorhydrin zu dem gewünschten Salz abreagieren läßt, dadurch gekennzeichnet, daß man eine optisch aktive Säure der allgemeinen Formel

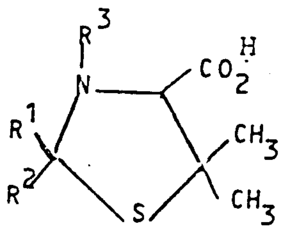

(II)

in der $R^1$, $R^2$, $R^3$ die oben angegebenen Bedeutungen haben, einsetzt.

Die optisch reinen Thiazolidin-4-carbonsäuren der allgemeinen Formel (II) können in an sich bekannter Weise, z. B. unter Verwendung von Brucin (GB-PS 585 413), vorzugsweise jedoch unter Verwendung von 1-Phenylpropanolamin (DE-OS 2 138 121) aus den racemischen Säuregemischen gewonnen werden. Ein bevorzugtes Verfahren zur Herstellung der optisch aktiven Salze gemäß der allgemeinen Formel (II) ist dadurch gekennzeichnet, daß man eine optisch aktive Säure der allgemeinen Formel (II), suspendiert in Wasser oder gelöst in einem organischen Lösungsmittel, vorlegt und je Mol dieser Säure 0,9 bis 1,2 Mol Trimethylamin bei 0 bis 30 °C hinzufügt.

Man kann aber auch umgekehrt vorgehen und die Lösung von Trimethylamin in Wasser oder vorzugsweise in einem organischen Lösungsmittel mit der Spaltsäure versetzen. Das als Zwischenprodukt gebildete Trimethylammoniumthiazolidin-4-carboxylat wird anschließend mit Epichlorhydrin bei Temperaturen von 5 bis 30 ° C in das gewünschte Salzpaar und dessen Diastereomeres überführt.

Ein besonderer Vorteile des erfindungsgemäßen Verfahrens liegt darin, daß die Salze der jeweiligen diastereomeren Salzpaare in organischen Lösungsmitteln beträchtliche Löslichkeitsunterschiede aufweisen und daher eine bequeme Trennung in Kristallisat und Mutterlauge gestatten. Nach den bisherigen Betrachtungen sind die Salze aus den D-Säure I mit dem L-(-)-Quab schwerer oder sogar wesentlich schwerer löslich als die diastereomeren D,D-Salze; das gleiche gilt natürlich für das Verhältnis der L,D-Salze zu den L,L-Salzen.

Wird die Reaktion in Wasser durchgeführt, muß das Wasser anschließend abgedampft und der Rückstand durch Behandeln mit einem organischen Lösungsmittel auskristallisiert werden. Zur Erhöhung der optischen Reinheit kann man das abgetrennte Salzpaar noch waschen, zweckmäßig mit Aceton, oder auch wie üblich umkristallisieren, z. B. aus Alkohol.

Eine bevorzugte Ausführungsform zur Herstellung des D,L-Salzpaares besteht darin, daß man 1 Mol der substituierten D-(+)-Thiazolidin-4- carbonsäure, suspendiert in Wasser oder in niederen Alkoholen oder Alkohol/Aceton-, Alkohol/Methylenchlorid- oder Alkohol/Essigestergemischen, vorlegt, bei Temperaturen von 0 bis 30 °C 0,9 bis 1,2 Mol Trimethylamin, vorzugsweise 1,0 bis 1,1 Mol Trimethylamin hinzufügt, anschließend in einem pH-Bereich von 7 - 9 mit 0,8 bis 1,2 Mol Epichlorhydrin versetzt und die Temperatur bei 5 - 30 °C, vorzugsweise bei 15 bis 20 °, hält.

Die Freisetzung des optisch aktiven Quabs 188 aus dem Salz kann wie üblich erfolgen, wobei das erfindungsgemäße Verfahren den verfahrenstechnisch besonderen Vorteil bietet, daß die Spaltsäuren, gemäß der allgemeinen Formel (II) schwer wasserlöslich sind und daher ohne irgendwelchen Destillations- und Extraktionsaufwand in hohen Ausbeuten zurückgewonnen werden können.

Beispielsweise kann man eine Lösung des Salzpaares in Wasser mit einer starken Mineralsäure wie Salzsäure versetzen, die ausgefallene Spaltsäure abtrennen und nach dem Trocknen wieder der Racematspaltung zuführen. Um das in hoher Ausbeute anfallende optisch aktive Quab 188 in hoher Reinheit zu erhalten, wird die wässrige Lösung mit einem mit Wasser nicht mischbaren organischen Lösungsmittel wie z. B. Methylenchlorid gewaschen und das optisch aktive Quab 188 durch Abdampfen des Wassers isoliert oder als wässrige Lösung zur Carnitin-Synthes eingesetzt.

Durch Änderung des Lösungsmittels lassen sich die Teilschritte dieses Verfahrensganges auch vertauschen, d. h. man versetzt eine Suspension aus Salzpaar und organischem Lösungsmittel wie z.B. Aceton oder Isopropanol mit Salzsäure und isoliert das unlösliche optisch aktive Quab 188 durch Abtrennung. Nach Eindampfen des organischen Lösungsmittel gewinnt man in hohen Ausbeuten die Spaltsäuren zurück. Dieses Verfahren findet bevorzugt bei der Rückgewinnung der optisch aktiven Spaltsäure aus der Mutterlauge der Salzpaarbildung Anwendung.

Daten D,L-Salzpaar

| | | | | | | | | | |
|---|---|---|---|---|---|---|---|---|---|
| Summenformel: | C15H29ClN04S | | | | | | | | |
| Molekurlargewicht: | | | | | | | | | 368,92 |
| Elementaranalyse: | ber.<br>gef. | C<br>C | 48,85<br>49,07 | H<br>H | 7,87<br>8.01 | Cl<br>Cl | 9,63<br>9,72 | N<br>N | 7,60<br>7,82 |
| Smp.: | 151 °C (nach Umkristallisation aus Isopropanol) | | | | | | | | |
| $[\alpha]_D^{20}$ = +31,4° (c=1, $H_2O$) | | | | | | | | | |

## Beispiele

### Beispiel 1

Zu einer Suspension aus 108,6 g (0,5 Mol) D-(+)-3-Formyl-2,2,5,5-tetramethyl-thiazolidin-4-carbonsäure in 240 ml Aceton tropfte man bei Raumtemperatur innerhalb 45 Minuten 119 ml einer 4,2 m (0,5 Mol) Trimethylaminlösung in Ethanol zu. Dabei stieg die Temperatur von 20 ° auf 26 °C an und es entstand eine klare Lösung. Es wurde noch 20 Minunten gerührt und anschließend 39,2 ml (0,5 Mol) Epichlorhydrin während 20 Minuten bei 25 °C zugetropft. Bei dieser Temperatur rührte man 48 Stunden, kühlte dann auf 5 °C und saugte das Salz aus (-)-3-Chlor-2-hydroxypropyltrimethylammonium und D-(+)-3-Formyl-2,2,5,5-tetramethylthiazolidin-4-carbonsäure ab. Nach Waschen mit Aceton und Trocknen im Vakuum erhielt man 51,0 g (27,6 %) Salzpaar.
$[\alpha]_D^{20}$ = +31,5 ° (c = 1, $H_2O$) Smp.: 150 bis 151 °C

### Beispiel 2

108,6 g (0,5 Mol) D-(+)-3-Formyl-2,2,5,5-tetramethylthiazolidin-4-carbonsäure wurden in einem Gemisch aus 70 ml Ethanol und 200 ml Essigester unter Rühren suspendiert und bei 15 - 20 °C 32,3 g (0,55 Mol) Trimethylamin während 40 Minuten zudosiert. Die klare Lösung versetzte man mit 39,2 ml (0,5 Mol)-Epichlorhydrin und rührte 2 Tage bei 25 °C. Das ausgefallene Salzpaar wurde abgenutscht, mit 150 ml Aceton gewaschen und im Vakuum getrochnet: 76,12 g (41,3 %).
$[\alpha]_D^{20}$ = +31,8 ° (C = 1, $H_2O$) Smp.: 146 bis 147°C

### Beispiel 3

34,8 g (0,59 Mol) Trimethylamin versetzte man bei -15 °C mit 200 ml Essigester und 70 ml Ethanol, erwärmte auf -5 °C und gab portionsweise 129,0 g (0,59 Mol) D-(+)-3-Formyl-2,2,5,5-tetramethylthiazolidin-4-carbonsäurehinzu. Anschließend wurde langsam auf Raumteperatur erwärmt und 46,3 ml (0,59 Mol) Epichlorhydrin zugetropft. Es wurde 4 Tage bei 25 °C gerührt, der entstandene Niederschlag abgesaugt, gewaschen und getrocknet. 76,06 g (34,7 %) Salzpaar.
$[\alpha]_D^{20}$ = +31,4 ° (c = 1, $H_2O$) Smp.: 150 °C

### Beispiel 4

217,3 g (1,0 Mol) D-(+)-3-Formyl-2,2,5,5-tetramethylthiazolidin-4-carbonsäure, 65,0 g (1,1 Mol) Trimethylamin und 70,4 ml (0,9 Mol) Epichlorhydrin wurden in 500 ml Isopropanol wie in Beispiel 2 beschrieben, zur Reaktion gebracht. Bei 15 °C Innentemperatur rührte man insgesamt 2 Tage. Der ausgefallene Kristallbrei wurde abgesaugt, mit insgesamt 250 ml Aceton gewaschen und getrocknet: 136,0 g (41 %) Salzpaar.
$[\alpha]_D^{20}$ = +32,9 ° (c = 1, $H_2O$)

Zur weiteren Reinigung suspendierte man den Feststoff in 500 ml Aceton und erhitzte unter kräftigem Rühren für 0,5 Stunden zum Rückfluß. Nach Abkühlen, Absaugen und Trocknen im Vakuum erhielt man 131,0 g (39,5 %) Salzpaar.
$[\alpha]_D^{20}$ = +32,0 ° (c = 1, $H_2O$) Smp.: 149 - 150 °C

### Beispiel 5

217,3 g (1,0 Mol) D-(+)-3-Formyl-2,2,5,5-tetramethylthiazolidin-4-carbonsäure, 65,0 g (1,1 Mol) Trimethylamin und 78,4 ml (1,0 Mol) Epichlorhydrin wurden in 600 ml Aceton,wie in Beispiel 2 beschrieben, zur Reaktion gebracht. Nach Absaugen des Feststoffes bei Raumtemperatur erhielt man 154,5 g (41,9 %) Salzpaar.
$[\alpha]_D^{20}$ = +34,6 ° (c = 1, $H_2O$) Smp.: 135 bis 137 °C

Beispiel 6

Herstellung von L-(-)-3-Chlor-2-hydroxypropyltrimethylammoniumchlorid [L-(-)-Quab 188]

Zu einer Lösung aus 130,1 g (0,353 Mol) Salzpaar $[\alpha]_D^{20}$ = +32,0 ° (C = 1, $H_2O$) in 300 ml Wasser tropfte man innerhalb 30 Minuten bei 10 °C 31 ml 36%ige Salzsäure und rührte noch 30 Minuten bei dieser Temperatur (pH-Wert der Suspension: 2,0). Die ausgefallene D-(+)-3,Formyl-2,2,5,5-tetramethylthiazolidin-4carbonsäure wurde abgesaugt, mit 100 ml Wasser gewaschen und bei 60 °C im Vakuum getrocknet: 72,3 g (94,3 %). Das wässrige Filtrat wurde zweimal mit 100 ml Methylenchlorid extrahiert. Nach Abtrennung und Einengen der organischen Phase sowie Trocknen des Rückstandes erhielt man weitere 2,0 g und somit insgesamt 74,3 g (97,0 %) D-(+)-3-Formyl-2,2,5,5-tetramethyl-thiazolidin-4-carbonsäure ($[\alpha]_D^{20}$ = +52,0 °; (C = 1, Ethanol) zurück.

Die wässrige Phase wurde bis zur Trockne bei 45 °C Badtemperatur im Vakuum eingeengt und zweimal mit je 100 ml Aceton versetzt und erneut eingeengt. Der verbliebene Rückstand wurde mit 150 ml Aceton digeriert, abgesaugt und bei 40 °C im Vakuum getrocknet: 65,1 g (98 %) L-(-)-Quab 188 als farblose Kristalle.
$[\alpha]_D^{20}$ = -28,7 ° (c = 1, $H_2O$) Smp.: 213 bis 214 °C

Nach erneutem Digerieren mit Aceton betrug der Drehwert
$[\alpha]_D^{20}$ = -29,9 ° (c = 1, $H_2O$).

Beispiel 7

Herstellung von L-(-)-3-Chlor-2-hydroxypropyltrimethylammoniumchlorid [L-(-)-Quab 188

Eine Suspension von 360,0 g (0,976 Mol) Salzpaar $[\alpha]_D^{20}$ = + 32,6 °, c = 1, $H_2O$) in 1,7 l Aceton kühlte man auf 5 °C und versetzte während 45 Minuten nacheinander mit 45,3 g 36 %iger (0,46 Mol) und 19,0 g (0,52 Mol) gasförmiger Salzsäure.
Anschließend wurde noch 30 Minuten bei 5 °C gerührt. Das ausgefallene Produkt wurde abgesaugt, mit 100 ml Aceton gewaschen und bei 40 °C im Vakuum getrocknet: 176,0 g (95,9 %) L-(-)-Quab 188.
$[\alpha]_D^{20}$ = -28,5 ° Smp.: 214 bis 215°

Das Filtrat wurde im Rotationsverdampfer eingeengt, der Rückstand mit 300 ml Wasser versetzt und teilweise eingeengt. Die so erhaltene Suspension saugte man ab, wusch mit 100 ml Wasser nach und erhielt nach dem Trocknen 207,4 g (97,8 %) D-(+)-3-Formyl-2,2,5,5-tetramethyl-thiazolidin-4-carbonsäure. Extraktion des wässrigen Filtrates mit Methylenchlorid erbrachte zusätzliche 3,9 g und somit insgesamt 211,3 g (99,6 %) optisch aktive Spaltsäure.

**Patentansprüche**

**1.** Optisch aktives Salz der allgemeinen Formel

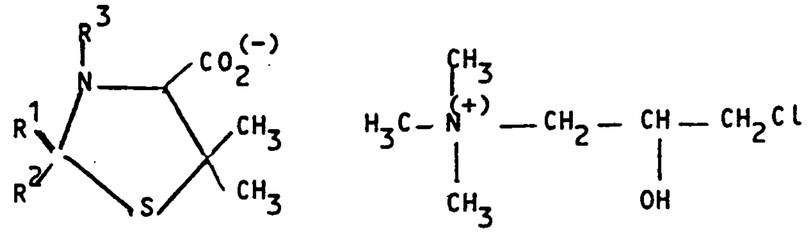 (I)

in der bedeuten
$R^1$, $R^2$, gleich oder verschieden: Wasserstoff, Alkyl mit 1 bis 8 C-Atomen, insbesondere Methyl und Ethyl,

$R^1$ oder $R^2$: Cycloalkyl mit 4 bis 11 C-Atomen
$R^3$ eine Acylgruppe, insbesondere Benzoyl, Tosyl, Nitrophenylsulfenyl, Acetyl oder Formyl.

**2.** Salz aus D-(+)-3-Formyl-2,2,5,5-tetramethylthiazolidin-4-carboxylat und L-(-)-3-Chlor-2-hydroxypropyltrimethylammonium

**3.** Salz aus L-(-)-3-Formyl-2,2,5,5-tetramethylthiazolidin-4-carboxylat und D-(+)-3-Chlor-2-hydroxypropyltrimethylammonium.

**4.** Verfahren zur Herstellung eines optisch aktiven Salzes gemäß Anspruch 1, bei dem man eine optisch aktive Säure mit Trimethylamin zu einem Trimethylammoniumsalz umsetzt, das man anschließend mit racemischem Epichlorhydrin zu dem gewünschten Salz abreagieren läßt,
dadurch gekennzeichnet, daß man eine optisch aktive Säure der allgemeinen Formel

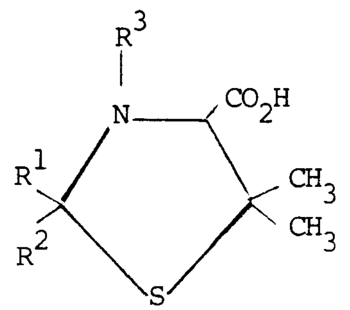

(II),

in der $R_1$, $R_2$ und $R_3$ die oben angegebenen Bedeutungen haben, einsetzt.

**5.** Verfahren zur Herstellung eines optisch aktiven Salzes gemäß Anspruch 1, dadurch gekennzeichnet, daß man eine optisch aktive Säure der allgemeinen Formel (II), suspendiert in Wasser oder gelöst in einem organischen Lösungsmittel, vorlegt, je Mol dieser Säure 0,9 bis 1,2 Mol Trimethylamin bei 0 bis 30 °C zusetzt, in einem pH-Bereich von 7 bis 9 0,8 bis 1,2 Mol Epichlorhydrin hinzufügt und nach erfolgter Umsetzung das gewünschte Salz abtrennt.

**6.** Verwendung der optisch aktiven Salze gemäß den Ansprüchen 1 bis 3 zur Herstellung von optisch aktivem 3-Chlor-2-hydroxypropyltrimethylammoniumchlorid, dadurch gekennzeichnet, daß man die Salze in an sich bekannter Weise mit Säuren spaltet.

## Claims

**1.** Optically active salts corresponding to the following general formula:

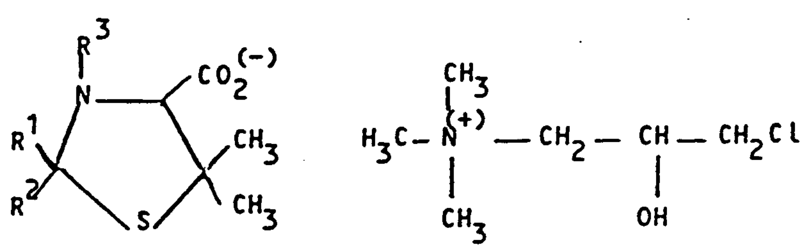

(I)

in which

| | |
|---|---|
| $R^1$ and $R^2$ | may be the same or different and represent hydrogen, $C_{1-8}$ alkyl, more particularly methyl and ethyl, |
| $R^1$ or $R^2$ | represents $c_{4-11}$ cycloalkyl, |
| $R^3$ | represents an acyl group, more particularly benzoyl, tosyl, nitrophenylsulfenyl, acetyl or formyl. |

2. A salt of D-(+)-3-formyl-2,2,5,5-tetramethylthiazolidine-4-carboxylate and L-(-)-3-chloro-2-hydroxypropyl trimethyl ammonium.

3. A salt of L-(-)-3-formyl-2,2,5,5-tetramethylthiazolidine-4-carboxylate and D-(+)-3-chloro-2-hydroxypropyl trimethyl ammonium.

4. A process for the production of the optically active salt claimed in claim 1, in which an optically active acid is reacted with trimethylamine to form a trimethyl ammonium salt which is then reacted off with racemic epichlorohydrin to form the desired salt, characterized in that an optically active acid corresponding to the following general formula

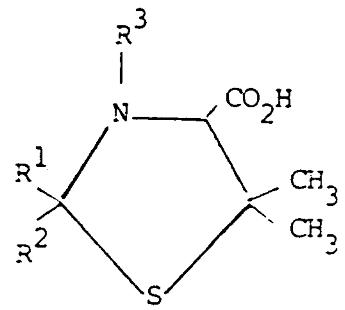

(II),

in which $R_1$, $R_2$ and $R_3$ are as defined above,
is used.

5. A process for the production of the optically active salt claimed in claim 1, characterized in that an optically active acid corresponding to general formula (II) is initially introduced suspended in water or dissolved in an organic solvent, 0.9 to 1.2 mol trimethylamine is added per mol of this acid at 0 to 30 ° C, 0.8 to 1.2 mol epichlorohydrin is added at a pH value of 7 to 9 and the desired salt is separated off on completion of the reaction.

6. The use of the optically active salts claimed in claims 1 to 3 for the production of optically active 3-chloro-2-hydroxypropyl trimethyl ammonium chloride, characterized in that the salts are resolved with acids by methods known <u>per</u> <u>se</u>.

**Revendications**

1. Sel optiquement actif de formule générale

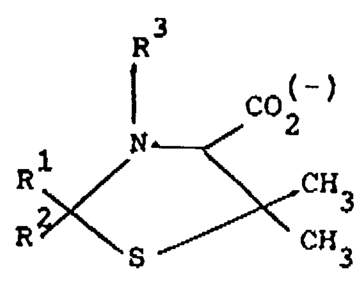

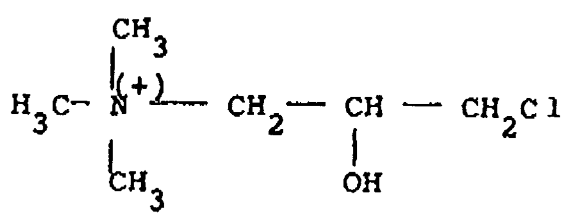

(I)

dans laquelle,

$R_1$ et $R_2$, identiques ou différents, signifient de l'hydrogène, un alcoyle ayant de 1 à 8 atomes de carbone, en particulier méthyle et éthyle,

$R_1$ ou $R_2$ : cyclo alcoyle ayant de 4 à 11 atomes de carbone.

$R_3$ signifie un radical acyle, en particulier benzoyle, tosyle, nitrophénylsulfényle, acétyle ou formyle.

2. Sel de D-(+)-3-Formyl-2,2,5,5-tétraméthyl-thiazolidine-4-carboxylate et de L-(-) 3-chloro-2-hydroxypro-

pyltriméthylammonium.

**3.** Sel de L-(-)-3-Formyl-2,2,5,5-tétraméthyl-thiazolidine 4-carboxylate et de D-(+)-3-chloro-2-hydroxypropyltrlméthylammonium.

**4.** Procédé d'obtention d'un sel optiquement actif selon la revendication l, dans lequel on transforme un acide optiquement actif avec la triméthylamine en un sel de triméthylammonium que l'on fait réagir ensuite avec de l'épichlorhydrine racémique en sel désiré, caractérise en ce que l'on met en oeuvre l'acide optiquement actif de formule générale

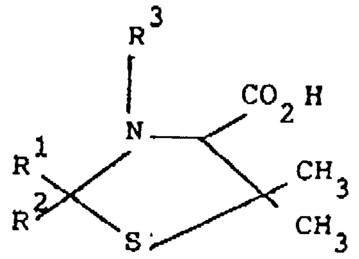

(II)

dans laquelle $R_1$, $R_2$ et $R_3$ possèdent les significations mentionnées ci-dessus.

**5.** Procédé d'obtention d'un sel optiquement-actif selon la revendication 1, caractérisé en ce que l'on introduit un acide optiquement-actif de formule générale (II) mis en suspension dans l'eau, ou dissout dans un solvant organique, on additionne par mol de cet acide 0,9 à 1,2 mol de triméthylamine entre 0 à 30° C, on ajoute dans une zone de pH allant de 7 à 9, 0,8 à 1,2 mol d'épichlorhydrine et on sépare après réaction effectuée le sel désiré.

**6.** Utilisation de sels optiquement-actifs conformément aux revendications 1 à 3 pour l'obtention du chlorure de 3-chloro 2-hydropropyltriméthylammonium optiquement-actif, caractérisé en ce que l'on dédouble les sels d'une manière connue en soi, par des acides.